# EUROPEAN PATENT APPLICATION

(11) **EP 2 937 050 A1**
(43) Date of publication of application: **28.10.2015**
(21) Application number: 12890528.8
(22) Date of filing: 19.12.2012
(51) Int. Cl.: A61B 17/80, A61B 17/84, A61B 17/68

(54) **MINIMALLY INVASIVE FIXATION PLATE FOR THE CORRECTION OF BUNIONS**

(71) Applicant: Industrias Medicas Sampedro S.A.S., Sabaneta, Antioquia (CO)
(72) Inventor: TORO, Mauricio, Sabaneta Antioquia (CO); BACCA, Gustavo, Sabaneta Antioquia (CO)
(74) Representative: Maldonado Jordan, Julia
(86) International application number: PCT/IB2012/057494
(87) International publication number: WO 2014/096905

(57) **Abstract**

The present invasion relates to a minimally invasive fixation plate for carrying out the correction of the deformation of the toes, known as Hallux Valgus, said plate is manufactured from a biocompatible material, preferably steel or titanium, which is also designed to facilitate the making of this minimally invasive surgery. The fixation plate comprises a superior portion or head with two double orifices with inner threads, where one of said threads has a larger diameter than the other, and a lower portion or bone insertion portion, wherein in a guide is attached through the larger thread which accomplished two functions: the first one is that it allows for manipulation of the plate and the second one is that it allows for the correct orientation of the drill bite and all the elements ensuring that the screw is blocked into the smaller thread which is straight and with a single entrance.

## Description

### TECHNICAL FIELD

The present invasion relates to a minimally invasive fixation plate for carrying out the correction of the deformation of the toes, known as Hallux Valgus, said plate is manufactured from a biocompatible material, preferably steel or titanium, which is also designed to facilitate the making of this minimally invasive surgery. The fixation plate comprises a superior portion or head with two double orifices with inner threads, where one of said threads has a larger diameter than the other, and a lower portion or bone insertion portion, wherein in a guide is attached through the larger thread which accomplishes two functions: the first one is that it allows for manipulation of the plate and the second one is that it allows for the correct orientation of the drill bite and all the elements ensuring that the screw is blocked into the smaller thread which is straight and with a single entrance.

Given the above, when surgery is carried out to correct Hallux Valgus with this plate, the pointed portion of the plate is inserted into the proximal portion of the metatarsus and the same is moved until the portion with the screws is aligned with the distal fragment. In this manner, the plate size and the osteotomy technique allow for the attachment to be made with a minimal incision and with the minimum compromise of soft tissue.

### BACKGROUND OF THE INVENTION

Currently, Hallux Valgus (commonly known as "bunion") represents a problem for patients, especially women, since this deformity affects both the foot's motor function as well as its aesthetic part. Because of this reason, it has been sought for many years how to repair or correct this deformity affecting the first segment of the digital metatarsus of the foot so as to improve both the physical aspect as well as the motor aspect and, in this way, help the patient throughout this process.

Hallux Valgus consists of the deviation of the first metatarsus where it leans towards the second metatarsus, which is generally due to malformations of the tendons and ligaments. During surgery for correcting Hallux Valgus, a cut is made (osteotomy) on the metatarsus by means of drill bites and saws, the bone is polished to correct any deformity on it.

Nonetheless, currently a plurality of procedures are carried out and implants are used to correct this deformity of the foot, which are generally effective but present the inconvenience that both the surgery and the recovery are long and painful for the patient, originated in a large measure by the trauma to sift tissue necessary for carrying out this surgery, because of which reason most of them have opted to endure this deformity hiding it with the use of certain shoes or with unconventional methods that do not allow for a relief or amelioration of said syndrome. In order to counteract this, there is a tendency towards minimally invasive techniques.

Additionally, it must be highlighted the fact that the procedures commonly carried out for correcting Hallux Valgus require a completely invasive surgery which affects the recovery of the patient, as previously mentioned, and it also has very high costs, which in some cases cannot be covered by the medical insurance or the patient himself and therefore, cannot be carried out directly affecting the patient.

Hence, in the state of the art there are a plurality of disclosures related to plates and similar devices which are currently used for correcting the deformity of the toes named Hallux Valgus, where said devices or plates are implanted on the patient's toe presenting the deformity and thus allowing, by means of force exerted on the bone in an opposite direction to Hallux Valgus, said problem can be corrected.

In this sense, document US 6.391.031 discloses a guide for an osteotomy and the method for use thereof, where said device is useful when carrying out a bi-plane cut in and osteotomy surgery where it is required to precisely redirect the bone or joint. This guide is used to mark a first cut and then placed in said cut. Similarly, the guide is then used to direct the second cut, where the use of this guide allows for these cuts to match perfectly, thus speeding up the healing process and making it less traumatic.

Nonetheless, the device disclosed by this document presents the disadvantage of requiring two cuts to the bone or joint of the patient, which cannot be considered as a minimally invasive surgery, which in turn leads to the aforementioned issues related to the patient's trauma and high recovery times.

On the other hand, document CN 201341977 discloses a medical apparatus for the correction of Hallux Valgus, which comprises a flexural metatarsal plate and a flexural phalanx plate which are overlapped, wherein the shape of the phalanx plate is inosculated with the inner side of phalanx, and the shape of the metatarsal plate is inosculated with the inner side of metatarsal, and the cross joint between said plates is provided with a rotation shaft which is movably connected, and a concave structure on the overlapping position of the metatarsal plate and the phalanx plate at the rotation shaft is inosculated with various deformities of the metatarsal for avoiding rubbing the patient's skin. The corrective appliance further comprises a soft pad which is arranged on the sides of the metatarsal plate and the phalanx plate, which are closer to skin, a rear bandage which is fixed on the back end of the metatarsal plate and a front bandage which is fixed on the front end of the phalanx plate. This device is appropriate to be used on patients having problems with Hallux Valgus and allows for a considerable improvement in the correction of said effect and allows the patients with a large problem of Hallux Valgus to avoid the long recovery which presents after surgery. Since the rotation shaft can bend with the joint of the metatarsus and the phalange, the Hallux Valgus corrector would not affect the patient's daily life at the same time as it corrects the problem.

Now, document WO 2011/086716 mentions a device to correct Hallux Valgus using a shape memory alloy, which can be easily used and exerts an adequate correcting force to correct in this way the Hallux Valgus or Hallux Varus, wherein said device comprises a correction plate formed from a shape memory alloy and attachment members or attaching the same, said attachment members each being formed of an elastic fabric bag, wherein said correcting plate is attached, from the inward direction of the hallux, to the foot body and to the hallux body respectively by the first attachment member and the second attachment member, and an opening is formed in the part of the correcting plate being in contact with the first metatarsophangeal joint so that the hallux is corrected by positioning outward using the periphery of the opening as a supporting point.

Finally, document US 2012101502 relates to surgical anchoring systems and methods that are used for correcting bone deformities, such a Hallux Valgus, where the correcting anchoring system includes am attachment system to anchor two or more sections of bone or other part of the body and a system for aligning a section with respect to another section, where said system requires perforation of the bone and anchoring to the adjacent section by means of a series of anchoring screws or means.

Nonetheless, the system disclosed in this anteriority corresponds to a device for invasive surgery requiring a series of alternate components and making it necessary to make cuts and incisions on the bones which represent a longer recovery time and more complex and traumatic procedures for the patient, which is undesirable and represents a disadvantage of the same for the afore mentioned reasons.

According to the above, it is evident for the skilled technician that there exists a need in the state of the art to design and implement a device to carry out minimally invasive surgery, wherein said device can be inserted in the patient's bone to keep it in place and ensure the correction of Hallux Valgus without the need of external elements for attachment, which would also reduce the costs of the procedure.

### BRIEF DESCRIPTION OF THE FIGURES

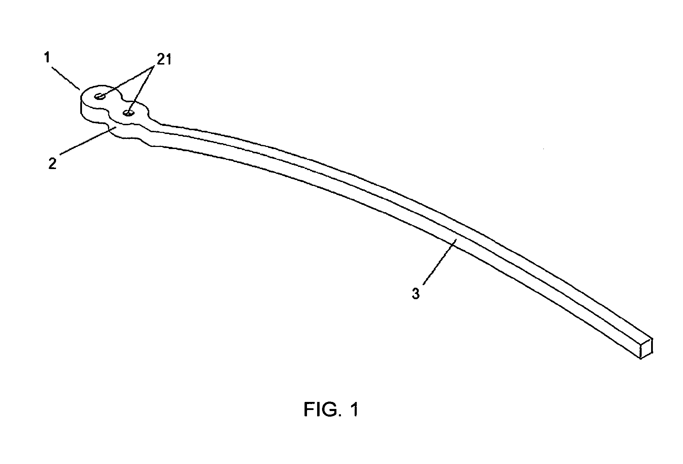

Figure 1 corresponds to a perspective view of the fixation plate of the present invention.

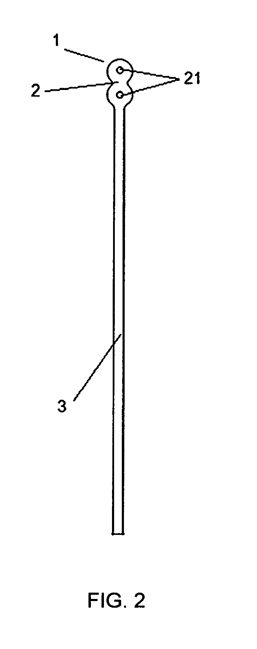

Figure 2 corresponds to a top view of the fixation plate of figure 1.
Figure 3 corresponds to a front view of the fixation plate of figure 1.

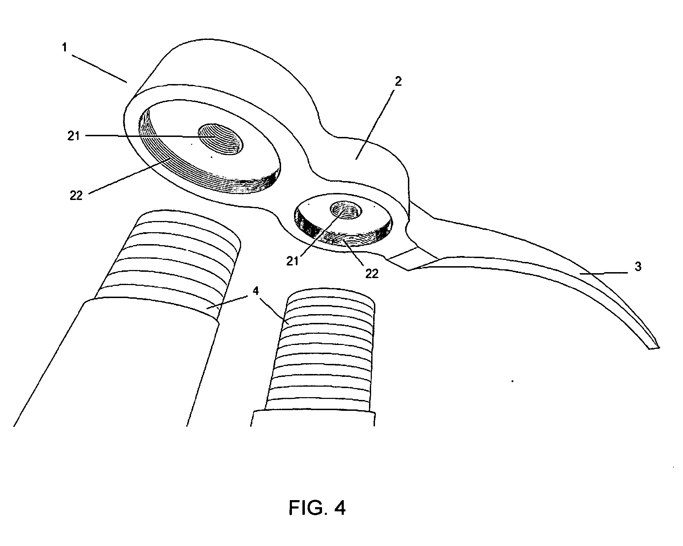

Figure 4 corresponds to a lower perspective view of the fixation plate of the present invention, where it is shown in detail the double thread of the upper portion thereof.

### DETAILED DESCRIPTION OF THE INVENTION

The minimally invasive fixation plate (1) for correcting Hallux Valgus of the present invention comprises the following components or parts:
- A head or upper portion (2) which counts with two internally threaded orifices (21, 22) that allow the manipulation of the plate (1) as well as the insertion of a screw for bone attachment and making the correction of Hallux Valgus; and
- A lower portion or insertion portion (3) which is inserted in the patient's bone.

Given the above, the present invention is directed towards a fixation plate (1) which corresponds to a single piece element comprising a body made up of a head or upper portion (2) and a lower portion or insertion portion (3), where the main characteristic thereof is that on the head (2) there is a double threaded system, where said thread corresponds to the inner surface of the orifices (21, 22).

In this way, the diameter of the hole (21) is a smaller diameter of the hole (22), since hole (22) is used for attachment of the plate (1) by means of a series of outer screws (4) which allow the plate (1) to attach to the bone and only require making a small incision on the patient, with a minimum compromise of soft tissues, and through each of the screws (4) the drill bit is passed and another screw is inserted (not shown) which is of a smaller diameter and it enters in the hole (21) in a precise way ensuring the plate and the outer elements thereof are installed in a successful way and it is possible to carry out the correction of Hallux Valgus.

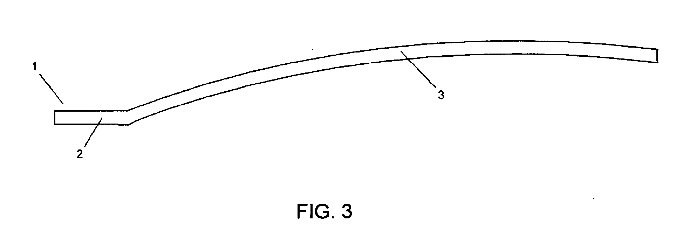

In one embodiment, the lower portion or insertion portion (3) has a length which is greater than the length to the head or the upper portion (2) and furthermore, it has a curved shape towards the outer portion of the foot, as shown in figure 3, so when it is installed on the metatarsus, an outward force from the patient's foot is excerted and thus the Hallux Valgus is corrected.

In a preferred embodiment of the invention, the fixation plate (1) was manufactured in a biocompatible material, preferably steel or titanium, which is also designed to facilitate the realization of this minimally invasive surgery.

Finally, when surgery is carried out for correcting Hallux Valgus with the plate (1) of the present invention, the pointed insertion portion (3) is inserted in the proximal portion of the metatarsus and the same is moved until the upper or head portion (2) which has the orifices (21, 22) for the screws is fully aligned with the distant fragment.

## Claims

1. A minimally invasive fixation plate for correcting Hallux Valgus, **characterized in that** it comprises:
• A head or upper portion (2) which counts with two internally threaded orifices (21, 22) that allow the manipulation of the plate (1) as well as the insertion of a screw for bone attachment and making the correction of Hallux Valgus; and
• A lower portion or insertion portion (3) which is inserted in the patient's bone, Wherein the fixation plate (1) is a single piece element which body is comprised by a head or upper portion (2) and the lower portion or insertion portion (3).

2. The fixation plate of claim 1, **characterized in that** the diameter of the hole (21) is smaller than the diameter of hole (22).

3. The fixation plate of claim 1 or 2, **characterized in that** the lower portion or insertion portion (3) has a length that is longer than the length of the head or upper portion (2) and also, they have a shaped that is curved towards the outer portion of the foot.

4. The fixation plate of any of the previous claims, **characterized in that** said plate (1) is manufactured in a biocompatible material, preferably steel or titanium.
